# EUROPEAN PATENT APPLICATION

(11) **EP 2 784 055 A1**
(43) Date of publication of application: **01.10.2014**
(21) Application number: 13305392.6
(22) Date of filing: 28.03.2013
(51) Int. Cl.: C07C 17/093

(54) **Fluoroform synthesis and uses thereof in the preparation of trifluoromethylated compounds**

(71) Applicant: Institut National des Sciences Appliquées de Rouen (INSA), 76801 St-Etienne du Rouvray Cedex (FR); Centre National de la Recherche Scientifique (CNRS), 75016 Paris (FR)
(72) Inventor: Jubault, Philippe, 76160 Preaux (FR); Pannecoucke, Xavier, 76150 Maromme (FR); Ivashkin, Pavel, 76130 Mont Saint Aignan (FR)
(74) Representative: Regimbeau

(57) **Abstract**

The present invention relates to a process to prepare fluoroform (CHF₃), notably radio labelled with fluorine-18, comprising a substitution reaction between CHAF₂ and a fluoride salt,
wherein A represents 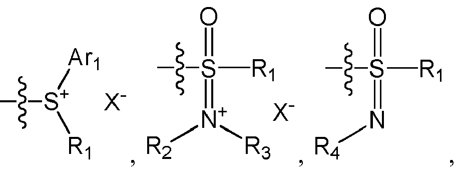 or 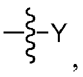 as well as a process to prepare a composition comprising copper-based or silver-based trifluoromethylated reagents using notably the fluoroform thus prepared and the use of a composition comprising copper-based trifluoromethylated reagents or a mixture comprising a copper salt and a composition comprising silver-based trifluoromethylated reagents as a trifluoromethylating agent, notably radio labelled with fluorine-18.

## Description

The present patent application relates to a process to prepare fluoroform, notably in a radiolabelled form, as well as the use thereof to prepare trifluoromethylated compounds via the preparation of copper-based or silver-based trifluoromethylated reagents.

Numerous pharmaceutical compounds comprise a trifluoromethyl moiety such as for example Fluoxetine (Prozac®), Mefloquine (Lariam®), Celecoxib (Celebrex®), Leflunomide (Arava®), Bicalutamide (Casodex®), or Cinacalcet (Sensipar®).

It could be interesting to obtain such compounds in a radiolabelled form with fluorine-18 since the unnatural radionuclide ¹⁸F has unique properties that are ideally suited to positron emission tomography (PET), one of the leading imaging modalities used in clinic, in drug discovery or in personalized medicine.

Several attempts of radiolabeling of aromatic CF₃ groups with ¹⁸F have been reported. These procedures give rise generally to the radiolabelled compounds with low specific activity (SA).

Carrier-added approaches based on the equilibration of ¹⁸F and ¹⁹F in the substrate [Ido et al. J. Labelled Comp. Radiopharm. 1979, 16, 153] or in the fluorinating agent [Josse et al. J. Labelled Comp. Radiopharm, 1997, 40 (5), 332] have intrinsically low specific activities due to the use of a large excess of non-labelled reactant and subsequent isotopic dilution.

At the same time, most of the no-carrier-added procedures reported so far demonstrate the SA orders of magnitude lower than what is expected for the methods without the isotopic dilution. H¹⁸F-Sb₂O₃ was used for the substitution in trichloromethylarene [Angelini et al. Chem. Commun. 1986, 924; Angelini et al. J. Labelled Comp. Radiopharm. 1990, 28, 1441]. Conversion of the labelled monofluorinated product into the trifluoromethylarene is probably accompanied by the isotopic exchange. Substitution did not proceed with a substrate bearing the amino group instead of nitro.

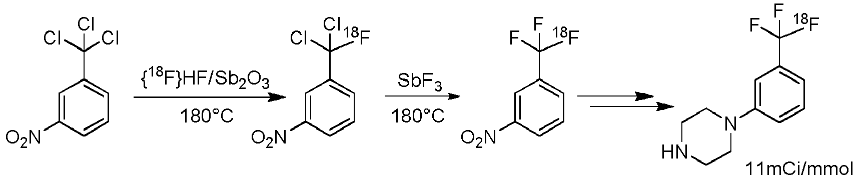

One-step conversion of difluorinated substrates into trifluoromethylarenes was accomplished using {¹⁸F}KF-K₂₂₂ [Hammadi and Crouzel J. Labelled Comp. Radiopharm. 1993, 33, 703; Das and Mukherjee Appl. Radiat. Isot. 1993, 44, 835] and {¹⁸F}NBu₄F [Prabhakaran et al. Bioorg. Med. Chem. 2007, 15, 1802] as fluorinating sources. Degradation of a substrate with the liberation of ¹⁹F⁻ is thought to be a reason for intensive isotopic dilution. Lowering the reaction temperature and the concentration of substrate was found to increase the specific activity (limited by the slow kinetics).

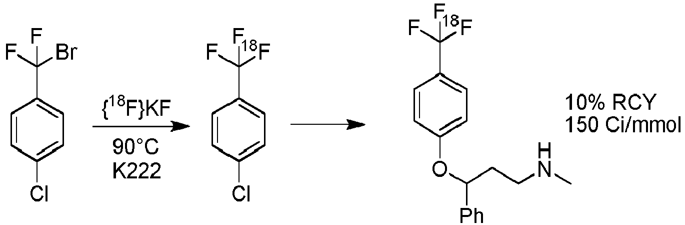

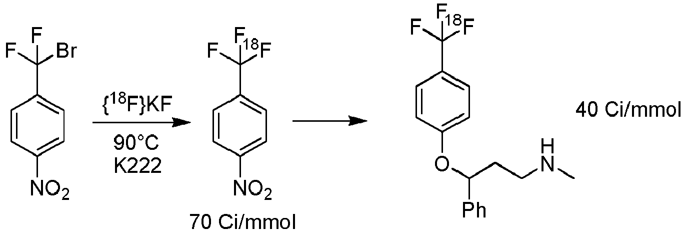

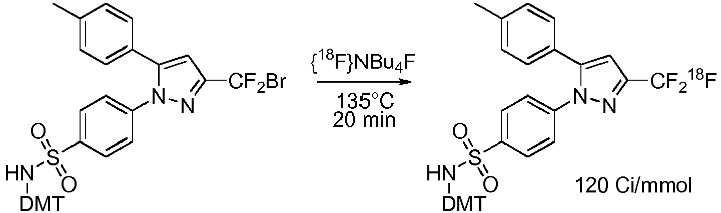

There exists thus a need for an improved method to introduce a radiolabelled CF₃ moiety on organic compounds, notably with improved kinetics, in order to obtain a high specific activity.

The present invention deals thus with an alternative method of trifluoromethylation in which the key step of formation of the CF₃ group proceeds with good yields and under very mild conditions and the CF₃-containing product is immediately removed from the reaction mixture thus minimizing the probability of isotopic dilution via generation of non-radioactive [¹⁹F]-fluoride and side-reactions.

The preparation of a trifluoromethylating agent ("CuCF₃") from fluoroform (CHF₃) and a cuprating agent was described by Grushin et al. [WO 2012/113726; Zanardi et al. J. Am. Chem. Soc. 2011, 133 (51), 20901], as well as the use thereof to prepare trifluoromethylated compounds from halogenated derivatives, boronic acids or α-haloketones [WO 2012/113726; Zanardi et al. J. Am. Chem. Soc. 2011, 133 (51), 20901; Tomashenko and Grushin Chem. Rev. 2011, 111, 4475; Novak et al. Angew. Chem. Int. Ed. 2012, 51, 7767; Novak et al. J. Am. Chem. Soc. 2012, 134 (39), 16167].

The development of a radio labelling method based on the chemistry of "CuCF₃" derived from fluoroform is of special interest, since both the synthesis of "CuCF₃" and its reactions with boronic acids and α-haloketones, for example, demonstrate fast kinetics that is crucial for the labelling with fast-decaying ¹⁸F. Moreover, an aromatic boronic acid functionality can be easily introduced into complex molecules and can therefore provide access to a very wide scope of [¹⁸F]-trifluoromethylated compounds. Trifluoromethylation of boronic acids described by Grushin et al. tolerates the presence of numerous functional groups, thus allowing introduce the [¹⁸F]-CF₃ group on a late stage of the synthesis and avoid the extensive use of protecting groups. Overall, these characteristics would result in minimization of the time needed for the radiosynthesis of [¹⁸F]-trifluoromethylated PET probes and therefore higher radiochemical yields.

For the successful implementation of the strategy described above one needs an efficient method of synthesis of fluoroform, in particular its [¹⁸F]-radiolabelled form from the usual ¹⁸F sources, such as [¹⁸F]-fluoride or [¹⁸F]F₂.

The authors of the present invention have thus developed a method to synthesize fluoroform from various sources of fluoride anion and a compound of formula CHAF₂, where A represents a good leaving group, such as a diarylsulfonium or an arylsulfoximinium. The reaction sequence developed by Grushin *et al.* can then be applied to the synthesis of {¹⁸F}-labelled trifluoromethylated compounds using no-carrier-added {¹⁸F}-fluoride. The authors of the present invention have also developed another trifluoromethylation process from fluoroform via the preparation of silver-based trifluoromethylated reagents.

The present invention relates thus to a process to prepare fluoroform (CHF₃), also called trifluoromethane, comprising a substitution reaction between CHAF₂ and a fluoride salt,
wherein A represents 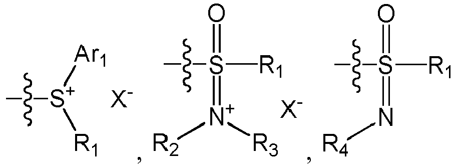 or 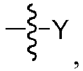 with:
■ X⁻ representing a non-nucleophile non-coordinating anion, such as BF₄⁻, PF₆⁻, SbF₆⁻, or B(Ar₂)₄⁻ with Ar₂ representing an aryl optionally substituted with one or several groups chosen from among (C₁-C₆)alkyl and (C₁-C₆)haloalkyl,
■ Y representing OSO₂R₅ or NR₆R₇R₈⁺ X⁻,
■ Ar₁ representing an aryl optionally substituted with one or several (C₁-C₆)alkyl groups,
■ R₁ representing a (C₁-C₆)alkyl group or an aryl optionally substituted with one or several (C₁-C₆)alkyl groups,
   where, when R₁ represents an optionally substituted aryl group, the aryl moieties of Ar₁ and of R₁ can be bound by a single bond,
■ R₂ and R₃ representing, independently of each other, a (C₁-C₆)alkyl group,
■ R₄ representing -OS(O)₂Ar₃ with Ar₃ representing an aryl optionally substituted with one or several groups chosen from among (C₁-C₆)alkyl and (C₁-C₆)haloalkyl,
■ R₅ representing a (C₁-C₆)alkyl or (C₁-C₆)haloalkyl group, said group being optionally substituted with (C₁-C₆)alkoxy or (C₁-C₆)haloalkoxy; or an aryl optionally substituted with one or several groups chosen from among halogen, (C₁-C₆)alkyl and (C₁-C₆)haloalkyl, and
■ R₆, R₇ and R₈ representing, independently of one another, a (C₁-C₆)alkyl, aryl heteroaryl, aryl-(C₁-C₆)alkyl or (C₁-C₆)alkyl-aryl group, or
(R₆ and R₇) or (R₇ and R₈) or (R₆ and R₈) forming a heterocycle with the nitrogen atom carrying them.

The term "halogen", as used in the present invention, refers to a fluorine, bromine, chlorine or iodine atom.

The term "(C₁-C₆)alkyl", as used in the present invention, refers to a straight or branched saturated hydrocarbon chain containing from 1 to 6 carbon atoms including, but not limited to, methyl, ethyl, n-propyl, iso-propyl, n-butyl, iso-butyl, sec-butyl, t-butyl, n-pentyl, n-hexyl, and the like.

The term "(C₁-C₆)haloalkyl", as used in the present invention, refers to a (C₁-C₆)alkyl group as defined above substituted with at least one halogen atom, and preferably by at least one fluorine atom. It can be in particular a trifluoromethyl group.

The term "(C₁-C₆)alkoxy", as used in the present invention, refers to a (C₁-C₆)alkyl group as defined above bound to the molecule via an oxygen atom, including, but not limited to, methoxy, ethoxy, n-propoxy, iso-propoxy, n-butoxy, iso-butoxy, sec-butoxy, t-butoxy, n-pentoxy, n-hexoxy, and the like.

The term "(C₁-C₆)haloalkoxy", as used in the present invention, refers to a (C₁-C₆)alkoxy group as defined above substituted with at least one halogen atom, and preferably by at least one fluorine atom. It can be in particular a trifluoromethoxy group.

The term "aryl", as used in the present invention, refers to an aromatic hydrocarbon group comprising preferably 6 to 10 carbon atoms and comprising one or more fused rings, such as, for example, a phenyl or naphtyl group. Advantageously, it is a phenyl group.

The term "aryl-(C₁-C₆)alkyl", as used in the present invention, refers to an aryl group as defined above bound to the molecule via a (C₁-C₆)alkyl group as defined above. In particular, it can be a benzyl group.

The term "(C₁-C₆)alkyl-aryl", as used in the present invention, refers to a (C₁-C₆)alkyl group as defined above bound to the molecule via an aryl group as defined above. In particular, it can be a tolyl group (CH₃Ph).

The term "heteroaryl" as used in the present invention refers to an aromatic group, preferably a 5- to 10-membered aromatic group, comprising one or more fused rings, in which the atoms of the ring(s) consist of one or more, advantageously 1 to 4, and more advantageously 1 or 2, heteroatoms, such as a nitrogen, oxygen or sulphur atom, the remainder being carbon atoms. A heteroaryl group can be notably thienyl, furanyl, pyrrolyl, etc.

The term "heterocycle" as used in the present invention refers to a saturated, unsaturated or aromatic monocycle or polycycle (comprising fused, bridged or spiro rings) comprising preferably 5 to 10, notably 5 or 6, atoms in the ring(s), in which the atoms of the ring(s) consist of one or more, advantageously 1 to 4, and more advantageously 1 or 2, heteroatoms, such as a nitrogen, oxygen or sulphur atom, the remainder being carbon atoms. A heterocycle can be notably piperidine, pyrrolidine, etc.

The term "(C₃-C₁₀)cycloalkyl", as used in the present invention, refers to a hydrocarbon ring having 3 to 10 carbon atoms, preferably, 3, 5, 6, 7 or 8, including, but not limited to, cyclopropyl, cyclopentyl, cyclohexyl and the like.

By "room temperature", is meant, in the framework of the present invention, a temperature comprised between 15 and 40°C, preferably between 20 and 30°C and notably of about 25°C.

The term "alkali metal", as used in the present invention, refers notably to Li, Na, K, Cs and Rb.

According to a preferred embodiment, the fluoride salt is N((C₁-C₆)alkyl)₄F or MF with M representing an alkali metal, and in particular it is N((C₁-C₆)alkyl)₄F. It can be in particular LiF, NaF, KF, CsF, RbF, NMe₄F or NBu₄F, notably KF, CsF or NBu₄F, and preferably NBu₄F, such as NBu₄F3H₂O.

When the fluoride salt is MF, and thus notably LiF, NaF, KF, CsF or RbF, such as KF or CsF, the substitution reaction can be performed in the presence of a crown ether, such as 18-crown-6.

The substitution reaction can be carried out in a solvent comprising a nitrile function, dichloromethane (DCM), dichloroethane (DCE), ethyl acetate (EtOAc), diethyl ether (Et₂O) or a mixture thereof. The solvent used for the substitution reaction is advantageously a solvent comprising a nitrile function such as acetonitrile (ACN), benzonitrile, butyronitrile, propionitrile or a mixture thereof. It can be in particular acetonitrile (ACN). Indeed, the use of a solvent comprising a nitrile function allows preventing degradation of CHAF₂ when it is a sulfonium, such a degradation being observed with other solvents, improving the yield in fluoroform and reducing unwanted side-reactions during the next step of preparation of copper-based trifluoromethylated reagents formed from fluoroform as described below.

Preferably, N((C₁-C₆)alkyl)₄F, and in particular NBu₄F and notably NBu₄F3H₂O, is used as fluoride salt, and ACN is used as solvent.

The substitution reaction can be performed at a temperature between 0 and 120°C, preferably between 0 and 50°C, notably between 20 and 30°C and in particular at room temperature.

According to a particular embodiment, the fluoride salt comprises fluorine-18 (isotope ¹⁸F).

Substituent A can be advantageously 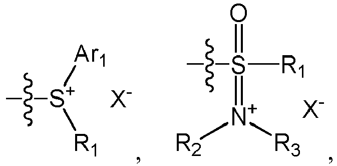 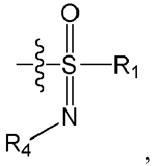 or 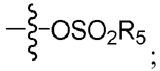 and in particular 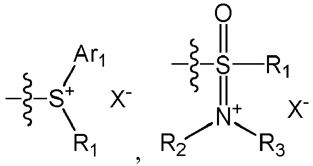 or 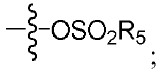 such as

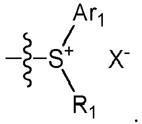

Advantageously, Ar₁ and R₁ represent, independently or each other, an aryl optionally substituted with one or several (C₁-C₆)alkyl groups, such as methyl groups.

R₂ and R₃ represent, independently of each other, a (C₁-C₆)alkyl group, such as methyl.

R₅ represents in particular a (C₁-C₆)alkyl or (C₁-C₆)haloalkyl group, such as a (C₁-C₆)alkyl or (C₁-C₆)fluoroalkyl group. It can be notably CF₃.

Substituent A can be chosen from among: 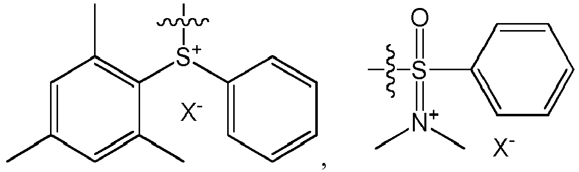 and 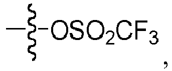 and in particular is 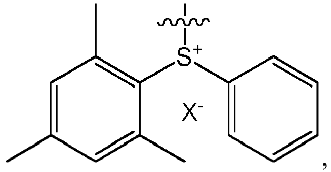 with X as defined previously.

X⁻ represents notably BF₄⁻ or B(Ar₂)₄⁻, notably B(Ar₂)₄⁻, with Ar₂ as defined previously and advantageously representing an aryl optionally substituted with one or several (C₁-C₆)haloalkyl groups, notably (C₁-C₆)fluoroalkyl groups such as CF₃. Ar₂ can be 3,5-bis(trifluoromethyl)-phenyl.

In particular, A can be: 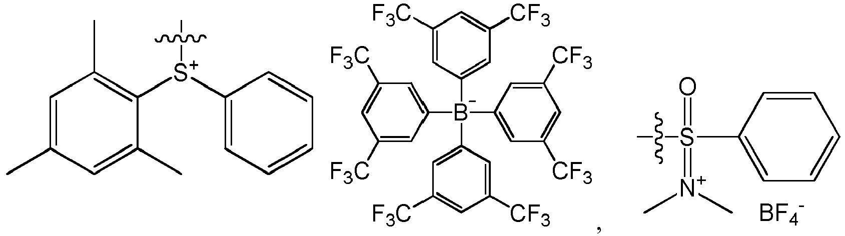 or

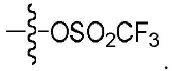

Advantageously, A is:

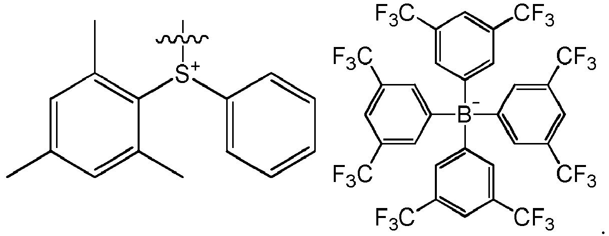

The molar ratio of CHAF₂ to the fluoride salt is not crucial for the substitution reaction. In general, the cheaper of these two compounds will be used in excess.

According to a particular embodiment, the process is carried out under an inert atmosphere, notably under argon or nitrogen.

The present invention relates also to a process to prepare a composition comprising copper-based trifluoromethylated reagents comprising the following steps:
(i) reacting a copper (I) salt and a base to obtain a cuprating agent, and
(ii) reacting the cuprating agent thus obtained with fluoroform prepared according to the process described above.

The composition comprising copper-based trifluoromethylated reagents obtained can then be used as a trifluoromethylating agent as described below. The structures of the copper-based trifluoromethylated reagents formed during step (ii) are not known but comprise a CuCF₃ moiety. These reagents can thus be named "CuCF₃".

Steps (i) and (ii) can be carried out successively or simultaneously. Preferably, step (i) is carried out before step (ii).

The copper (I) salt is advantageously chosen from among CuCl, CuBr, CuI, CuCN, (CuOSO₂CF₃)˙C₆H₆ and CuO((C₁-C₆)alkyl). Preferably it is CuCI.

The base is advantageously an alkali metal (C₁-C₆)alkoxide, such as a potassium, sodium or lithium (C₁-C₆)alkoxide. It can be in particular *t*BuOK.

Advantageously, at least 2 moles of base are used for 1 mole of copper (I) salt. Molar ratios copper (I) salt / base comprised between 1:2 and 1:4 have been tested leading to satisfying results. The best results are obtained with a molar ratio copper (I) salt / base of 1:3.

A ligand can also be used in step (i), such as 1,10-phenanthroline.

According to a particular embodiment, step (i) is performed under sonication in order to improve the reaction between the copper (I) salt and the base.

The process is carried out in a solvent which can be DMF, DMSO, DMI (1,3-dimethyl-2-imidazolidinone), NMP (N-methyl-2-pyrrolidone), N-methylpyrrolidine, or a mixture thereof; and preferably is DMF.

Advantageously, the process is performed at room temperature.

According to a particular embodiment, the process is carried out under an inert atmosphere, notably under nitrogen or argon.

An acid or a borate can be added after step (ii) in order to neutralize the excess of the base used in the process (e.g. *t*BuOK) or the excess of the cuprating agent formed during step (i) (e.g. KCu(O*t*Bu)₂ if *t*BuOK is used as a base).

The acid is preferably a mild acid. It can be hydrogen fluoride or a derivative or complex thereof, such as triethylamine trihydrofluoride (NEt₃·3HF also called TREAT-HF). Advantageously, about 1/n mole of acid is used for 1 mole of base, n representing the number of protons that can be donated by the acid. For example, in the case of TREAT-HF (NEt₃·3HF), the acid comprises 3 molecules of HF complexed with triethylamine. Three protons can thus be donated and about 1/3 mole of TREAT-HF is advantageously used for 1 mole of base.

The borate is notably B(OR₉)(OR₁₀)(OR₁₁) with R₉, R₁₀ and R₁₁, identical or different, preferably identical, representing a(C₁-C₆)alkyl group, such as a methyl. It is advantageously trimethyl borate (B(OMe)₃).

A filtration step can also be performed at the end of the process to remove the solids which have precipitated during the process.

The final composition obtained at the end of the process is notably in the form of a solution of the copper-based trifluoromethylated reagents, in particular in the solvent used for the reaction as defined previously. The composition can comprise other compounds than the copper-based trifluoromethylated reagents, such as unreacted starting materials or by-products.

The process to prepare the composition comprising copper-based trifluoromethylated reagents comprises the following steps:
(i) reacting a copper (I) salt and a base to obtain a cuprating agent,
(ii) reacting the cuprating agent thus obtained with fluoroform prepared according to the process described previously, and
(iii) optionally adding an acid or a borate B(OR₉)(OR₁₀)(OR₁₁).

According to a preferred embodiment, the process to prepare the composition comprising copper-based trifluoromethylated reagents comprises the following steps, performed advantageously in DMF:
(i) reacting CuCl and *t*BuOK to obtain a cuprating agent (KCu(O*t*Bu)₂),
(ii) reacting the cuprating agent thus obtained with fluoroform prepared according to the process described above, and
(iii) optionally adding TREAT-HF or B(OMe)₃.

More details concerning the process of preparation of the copper-based trifluoromethylated reagents are described in WO 2012/113726, the teaching of which is incorporated herein by reference.

According to a particular embodiment, the fluoroform used in this process is radiolabelled with fluorine-18 and allows the formation of copper-based trifluoromethylated reagents radio labelled with fluorine-18.

The present invention relates thus also to a composition comprising copper-based trifluoromethylated reagents radiolabelled with fluorine-18 obtainable by the process described above performed with a fluoroform radio labelled with fluorine-18.

The present invention relates also to the use of a composition comprising copper-based trifluoromethylated reagents radiolabelled with fluorine-18, obtainable by the process described above performed with fluoroform radiolabelled with fluorine-18, as a radiolabelled trifluoromethylating agent.

According to a particular embodiment, such a composition comprising copper-based trifluoromethylated reagents radio labelled with fluorine-18 is used in the presence of a silver (I) salt such as Ag(OTf), Ag(OTs), AgSbF₆, AgNO₃ or Ag(OAc), in particular Ag(OTf).

The composition comprising copper-based trifluoromethylated reagents radiolabelled with fluorine-18 is able in particular to react with a halogenated derivative, a boronic acid derivative, a boronic ester derivative or a boronate derivative to replace the halogen atom, the boronic acid function (B(OH)₂), the boronic ester function or the boronate function with a trifluoromethyl group.

The term "boronic ester function", as used in the present invention, refers to the function -B(OR₁₂)(OR₁₃) with R₁₂ and R₁₃ advantageously representing, independently of one another, a (C₁-C₆)alkyl or R₁₂ and R₁₃ forming together a linear or branched saturated hydrocarbon chain comprising 2 to 10 carbon atoms, such as a chain -CRₐR_{b}-CR_{c}R_{d}- with Rₐ, R_{b}, R_{c} and R_{d} representing, independently of each other, a (C₁-C₄)alkyl group, on the condition that the chain CRₐR_{b}-CR_{c}R_{d}- does not comprise more than 10 carbon atoms.

The term "boronate function", as used in the present invention, refers to the function -B⁻(OR₁₄)(OR₁₅)(OR₁₆) X₁⁺ with X₁⁺ representing Li⁺, Na⁺, K⁺ or N((C₁-C₆)alkyl)₄⁺ such as NBu₄⁺, and preferably Li⁺, and R₁₄, R₁₅ and R₁₆ advantageously representing, independently of each other, a (C₁-C₆)alkyl group or forming together a trivalent saturated hydrocarbon chain comprising 2 to 10 carbon atoms, notably 5 or 6 carbon atoms, such as 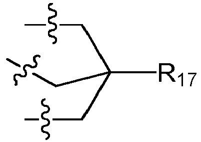 with R₁₇ representing a hydrogen atom or a (C₁-C₆)alkyl group, notably a methyl or ethyl group.

The derivatives which can be trifluoromethylated by the composition comprising copper-based trifluoromethylated reagents radiolabelled with fluorine-18 above mentioned are notably:
- a halogenated aromatic derivative of formula A₁-Hal₁ with Hal₁ representing a halogen atom such as I or Br, and A₁ representing an optionally substituted aryl or an optionally substituted heteroaryl,
- an α-haloketone of formula A₂-C(=O)CH₂-Hal₂ with Hal₂ representing a halogen atom such as Cl or Br, and A₂ representing an optionally substituted aryl, an optionally substituted heteroaryl, an optionally substituted (C₃-C₁₀)cycloalkyl, an optionally substituted heterocycle or an optionally substituted (C₁-C₆)alkyl,
- a boronic acid derivative of formula A₃-B(OH)₂ with A₃ representing an optionally substituted aryl or an optionally substituted heteroaryl,
- a boronic ester derivative of formula A₄-B(OR₁₂)(OR₁₃) with A₄ representing an optionally substituted aryl or an optionally substituted heteroaryl and R₁₂ and R₁₃ as defined above, or
- a boronate derivative of formula A₅-B⁻(OR₁₄)(OR₁₅)(OR₁₆) X₁⁺ with A₅ representing an optionally substituted aryl or an optionally substituted heteroaryl, and X₁⁺, R₁₄, R₁₅ and R₁₆ as defined.

The trifluoromethylation reaction can be performed in the presence of a silver (I) salt such as Ag(OTf), Ag(OTs), AgSbF₆, AgNO₃ or Ag(OAc), notably AgOTf, in particular when the derivative to be trifluoromethylated is a boronic acid derivative. In this case, it is not necessary to treat the composition comprising copper-based trifluoromethylated reagents radiolabelled with fluorine-18 with an acid or a borate, as described above, since the boronic acid can act as a neutralising agent.

The present invention relates also to a process to prepare a composition comprising silver-based trifluoromethylated reagents comprising the following steps:
(a) reacting a silver salt and a base to obtain a silver reagent, and
(b) reacting the silver reagent thus obtained with fluoroform.

The composition comprising silver-based trifluoromethylated reagents obtained can then be used in a trifluoromethylating reaction in the presence of a copper salt as described below. The structures of the silver-based trifluoromethylated reagents formed during step (b) are not known but comprise an AgCF₃ moiety. These reagents can thus be named "AgCF₃".

Steps (a) and (b) can be carried out successively or simultaneously. Preferably, step (a) is carried out before step (b).

The silver (I) salt is advantageously chosen from among AgOSO₂CF₃ (Ag(OTf)), AgOSO₂C₆H₄CH₃ (AgOTs), AgOSO₂CH₃, AgOAc, Ag OCOCF₃, AgCN, AgSbF₆, AgBF₄ and AgOC(CF₃)₃. Preferably it is Ag(OTf).

The base is advantageously an alkali metal (C₁-C₆)alkoxide, such as a potassium, sodium or lithium (C₁-C₆)alkoxide. It can be in particular *t*BuOK.

Advantageously, at least 1.5 moles, in particular between 1.5 and 4 moles, notably between 2 and 3 moles, such as about 2 moles, of base are used for 1 mole of silver salt.

According to a particular embodiment, step (a) is performed under sonication in order to improve the reaction between the silver salt and the base.

The process is carried out in a solvent which can be DMF, DMSO, DMI (1,3-dimethyl-2-imidazolidinone), NMP (N-methyl-2-pyrrolidone), N-methylpyrrolidine, or mixtures thereof; and preferably is DMF.

Advantageously, the process is performed at room temperature.

According to a particular embodiment, the process is carried out under an inert atmosphere, notably under nitrogen or argon.

An acid or a borate can be added after step (b) in order to neutralize the excess of the base used in the process (e.g. *t*BuOK) or the excess of the silver agent formed during step (a).

The acid is preferably a mild acid. It can be hydrogen fluoride or a derivative or complex thereof, such as triethylamine trihydrofluoride (NEt₃·3HF also called TREAT-HF). Advantageously, about 1/n mole of acid is used for 1 mole of base, n representing the number of protons that can be donated by the acid. For example, in the case of TREAT-HF (NEt₃·3HF), the acid comprises 3 molecules of HF complexed with triethylamine. Three protons can thus be donated and about 1/3 mole of TREAT-HF is advantageously used for 1 mole of base.

The borate is notably B(OR₉)(OR₁₀)(OR₁₁) with R₉, R₁₀ and R₁₁, identical or different, preferably identical, representing a (C₁-C₆)alkyl group, such as a methyl. It is advantageously trimethyl borate (B(OMe)₃).

A filtration step can also be performed at the end of the process to remove the solids which have precipitated during the process.

The final composition obtained at the end of the process is notably in the form of a solution of the silver-based trifluoromethylated reagents, in particular in the solvent used for the reaction as defined previously. The composition can comprise other compounds than the silver-based trifluoromethylated reagents, such as unreacted starting materials or by-products.

The process to prepare the composition comprising silver-based trifluoromethylated reagents comprises the following steps:
(a) reacting a silver salt and a base to obtain a silver reagent,
(b) reacting the silver reagent thus obtained with fluoroform, notably prepared according to the process described previously, and
(c) optionally adding an acid or a borate B(OR₉)(OR₁₀)(OR₁₁).

According to a preferred embodiment, the process to prepare the composition comprising silver-based trifluoromethylated reagents comprises the following steps, performed advantageously in DMF:
(a) reacting Ag(OTf) and *t*BuOK to obtain a silver reagent,
(b) reacting the silver reagent thus obtained with fluoroform, notably prepared according to the process described above, and
(c) optionally adding TREAT-HF or B(OMe)₃.

The fluoroform used in this process is advantageously prepared according to the process described previously. According to a particular embodiment, the fluoroform is radiolabelled with fluorine-18 and allows the formation of trifluoromethyl silver reagents radio labelled with fluorine-18.

The present invention relates thus also to a composition comprising silver-based trifluoromethylated reagents, notably radiolabelled with fluorine-18, obtainable by the process described above.

The present invention relates also to the use of a mixture comprising:
- the composition comprising silver-based trifluoromethylated reagents obtainable by the process described above, notably radiolabelled with fluorine-18, and
- a copper salt,
   as a trifluoromethylating agent.

The copper salt can be in particular:
- a copper (I) salt such as CuCl, CuBr, CuI, Cu(CH₃CN)₄PF₆, Cu(CH₃CN)₄OTf, CuOAc, CuCN, or (CuOTf)₂·C₆H₆, or
- a copper (II) salt such as Cu(OTf)₂, Cu(OAc)₂, Cu(OCOCF₃)₂ or copper (II) acetylacetonate.

Particularly, the copper salt will be CuCl or Cu(OTf)₂.

The mixture above-mentioned is able in particular to react with a halogenated derivative, a boronic acid derivative, a boronic ester derivative or a boronate derivative to replace the halogen atom, the boronic acid function (B(OH)₂), the boronic ester function or the boronate function with a trifluoromethyl group.

The derivatives which can be trifluoromethylated by the mixture above mentioned are notably:
- a halogenated aromatic derivative of formula A₁-Hal₁ as defined previously,
- an α-haloketone of formula A₂-C(=O)CH₂-Hal₂ as defined previously,
- a boronic acid derivative of formula A₃-B(OH)₂ as defined previously,
- a boronic ester derivative of formula A₄-B(OR₁₂)(OR₁₃) as defined previously, or
- a boronate derivative of formula A₅-B⁻(OR,₄)(OR₁₅)(OR₁₆) X₁⁺ as defined previously.

The present invention relates also to a method of imaging by positron emission tomography (PET) involving a compound radiolabelled with fluorine-18 (and more particularly a compound comprising a trifluoromethyl group radiolabelled with fluorine-18) by means of:
- a composition comprising copper-based trifluoromethylated reagents radiolabelled with fluorine-18 obtainable by the process described previously, or
- a mixure of a copper salt and a composition comprising silver-based trifluoromethylated reagents radiolabelled with fluorine-18 obtainable by the process described previously.

The present invention is illustrated by the following non-limitative examples.

### EXAMPLES

The following abbreviations have been used in the examples:

| | |
|---|---|
| Ac | Acetyl (COCH₃) |
| ACN | : Acetonitrile |
| DCM | : Dichloromethane |
| DMF | : Dimethylformamide |
| ESI | : Electrospray ionisation |
| HPLC | : High Performance Liquid Chromatography |
| HRMS | : High-resolution mass spectra |
| NMR | : Nuclear Magnetic Resonance |
| rt | : Room temperature |
| TREAT-HF | : Triethylamine trihydro fluoride |
| Tf | : Trifluoromethanesulfonyl (SO₂CF₃) |
| Ts | 4-Methylbenzenesulfonyl (SO₂C₆H₄CH₃) |

NMR spectra were recorded on Bruker DPX 300. ¹⁹F NMR spectra were acquired with the relaxation time parameter set to 5 seconds. High-resolution mass spectra (HRMS) were recorded on Waters LCT Premier.

ACN, PhCN, EtCN, PrCN, and DCM were distilled from CaH₂ and stored above the MS 4Å. DMF (Acros, extra-dry), CuCl (Aldrich, ≥99.99%), tBuOK (Alfa Aesar), CHF₃ (Apollo Scientific), NBu₄F·3H₂O (Acros), AgOTf (Aldrich, ≥99%) and other reagents were used without purification.

### 1. Synthesis of compounds of formula CHAF₂

Compound **1:** (modification of the known method described in Prakash et al. Org. Lett. 2007, 9(10),1863)

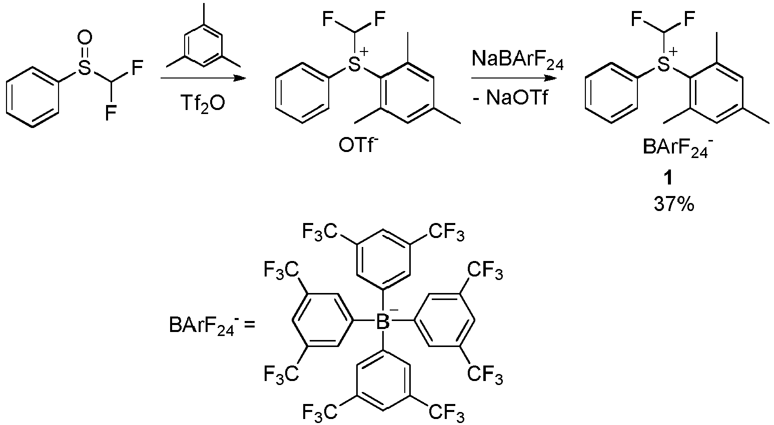

Triflic anhydride (0.8 mmol, 135 µL) was added dropwise at 0°C to a stirred solution of difluoromethyl phenyl sulfoxide (0.8 mmol, 140 mg) and mesytilene (1.2 mmol, 144 mg) in Et₂O (8 mL). After stirring for 7 min, the solvent was removed, the residual red oil was washed with Et₂O (5 mL) at 0°C and the solvent was removed again. Solid NaBArF₂₄ (0.4 mmol, 354 mg) was added followed by DCM (4 mL). After stirring for 1-2 min at 0°C, the mixture was diluted with toluene (10 mL) and filtered. The filtrate was diluted with petroleum ether (200 mL). The resulting precipitate was recrystallized from the mixture of DCM and petroleum ether at room temperature to give pure compound **1**·DCM as colorless crystals (343 mg, 38% from difluoromethyl phenyl sulfoxide). Compound **1**·DCM is a bench-stable, non-hygroscopic crystalline compound. Its composition was confirmed by the single-crystal X-Ray analysis. Compound **1** free of DCM can be obtained upon drying of the ground crystals in high vacuum or via re-evaporation with an appropriate solvent such as chloroform. Both **1** and **1**·DCM can be used for the synthesis of CHF₃ with identical results.
HRMS (ESI+): 279.1021 (calculated for C₁₆H₁₇F₂S: 279.1019).
HRMS (ESI-): 863.0662 (calculated for C₃₂H₁₂F₂₄B: 863.0649).

### Compounds 2 and 3:

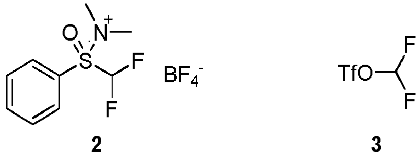

N,N-dimethyl-S-difluoromethyl-S-phenylsulfoximinium tetrafluoroborate **2** [G.K.S. Prakash et al.; Journal of Fluorine Chemistry 2011, 132, 792] and difluoromethyl trifluoromethanesulfonate **3** [V.V. Levin et al.; Journal of Fluorine Chemistry 2009, 130, 667] were prepared according to the known methods.

### 2. Synthesis of CHF₃ from compounds of formula CHAF₂ and fluoride salts Example from compound 1 and NBu₄FH₂O:

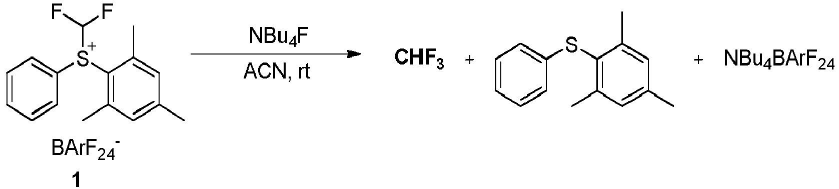

NMR tube containing 1 bead of non-activated molecular sieves (4Å) was sealed with a septum. Solution of tetrabutylammonium fluoride trihydrate (0.007 mmol, 2.2 mg) in 0.2 mL of anhydrous ACN was introduced into the tube, followed by 1.5 mL of pure ACN. Solution of **1** (0.006 mmol, 6.9 mg) in 0.2 mL of anhydrous ACN was added to the tube. The tube was carefully filled with pure ACN until the air is completely displaced. The tube was turned upside down about 30 times to ensure the mixing of its contents with the help of an MS 4Å bead. The resulting homogeneous sample was immediately analysed by ¹⁹F NMR. Signals were calibrated against the peak of BArF₂₄ anion. Alternatively, 1-bromo-3,5-difluorobenzene was added to the solution of tetrabutylammonium fluoride trihydrate as an internal standard. Consistent results were obtained with and without the use of an internal standard.

The results obtained with other compounds of formula CHAF₂, fluoride salts, solvents, according to the above procedure are given in the table below.

| **CHAF₂** | **Fluoride salt (equiv.)** | **Solvent** | **Conversion of CHAF₂ (%)** | **Yield of CHF₃ % from CHAF₂ (% from fluoride)** |
|---|---|---|---|---|
| **1** | NBu₄F_{˙}3H₂O (1.2) | ACN | 100 | 89 |
| **1** | NBu₄F_{˙}3H₂O (0.2) | ACN | 32 | 20(100) |
| **1** | NBu₄F_{˙}3H₂O (1.2) | ACN-d3 | 100 | 92* |
| **1** | NBu₄F_{˙}3H₂O (1.2) | ACN-H₂O (2%) | 100 | 9 |
| **1** | NBu₄F_{˙}3H₂O (1.2) | EtCN | 100 | 78 |
| **1** | NBu₄F_{˙}3H₂O (0.4) | EtCN | 53 | 42(100) |
| **1** | NBu₄F_{˙}3H₂O (1.2) | PrCN | 100 | 76 |
| **1** | NBu₄F_{˙}3H₂O (1.2) | PhCN | 100 | 80 |
| **1** | NBu₄F_{˙}3H₂O (0.9) | PhCN-ACN (3:1) | 88 | 62 (69) |
| **1** | NBu₄F_{˙}3H₂O (0.8) | DCM | 83 | 44 (55) |
| **1** | NBu₄F_{˙}3H₂O (0.5) | DCE | 51 | 28 (56) |
| **1** | KF (2) | ACN | 20 | 12 |
| **1** | KF(2) + 18-crown-6 (3) | ACN | 100 | 81 |
| **1** | CsF(1.2) | ACN | 62 | 30 |
| **2** | NBu₄F_{˙}3H₂O (1.2) | ACN | 100 | 55 |
| **3** | NBu₄F_{˙}3H₂O (1.2) | ACN | 100 | 30 |

| | | | | |
|---|---|---|---|---|
| * ratio of CHF₃:CDF₃ =1.5:1.0 | | | | |

### 3. Trifluoromethylation of boronic acids in the presence of TREAT-HF

### Synthesis of 2-benzyloxy-6-trifluoromethylnaphthalene:

CuCl (0.5 mmol, 50 mg) and tBuOK (1.5 mmol, 170 mg) were placed into a 10-mL flask equipped with a stirring bar. The flask was sealed with a septum, flushed with nitrogen. DMF (5.0 mL) was added and the resulting colorless mixture was stirred for 20 min. A balloon filled with nitrogen was attached. Excess of CHF₃ was introduced (balloon is slightly inflated). In three minutes TREAT-HF (0.5 mmol, 81 µL) was added.

2.0 mL of the above solution (theoretically containing 0.2 mmol CuCF₃) was added to the stirred solution of 6-benzyloxynaphthalene-2-boronic acid (0.4 mmol, 111 mg) in 1.0 mL of DMF. AgOTf (0.4 mmol, 103 mg) in DMF (1.0 mL) was added. After stirring in air for 10 min the mixture was diluted with 1M HCl, extracted with EtOAc (3×5 mL). The combined extracts were washed with brine, dried over MgSO₄ and evaporated to give the crude product of trifluoromethylation. Yield of 2-benzyloxy-6-trifluoromethylnaphthalene with respect to CuCF₃ used was 84% by ¹⁹F NMR (trifluorotoluene as an internal standard). The product was purified via column chromatography (dichloromethane - petroleum ether 10:90) to give 46 mg (77%) as a colorless solid.

In a parallel run 2.0 mL of CuCF₃ solution was added to the stirred solution of 6-benzyloxynaphthalene-2-boronic acid (0.4 mmol, 111 mg) in DMF (2.0 mL), without addition of a silver salt. After stirring in air for 10 min the mixture was quenched as described above and analysed by ¹⁹F NMR. The yield of 2-benzyloxy-6-trifluoromethylnaphthalene with respect to CuCF₃ used was 15% (trifluorotoluene as an internal standard).

### 4. Trifluoromethylation of boronic acids in the presence of B(OMe)₃

### Synthesis of 2-benzyloxy-6-trifluoromethylnahthalene:

CuCl (0.5 mmol, 50 mg) and tBuOK (1.5 mmol, 170 mg) were placed into a 10-mL flask equipped with a stirring bar. The flask was sealed with a septum, flushed with nitrogen. DMF (5.0 mL) was added and the resulting colorless mixture was stirred for 20 min. A balloon filled with nitrogen was attached. Excess of CHF₃ was introduced (balloon is slightly inflated). After stirring for 3 minutes trimethyl borate (2.5 mmol, 280 µL) was added.

2.0 mL of the above solution (theoretically containing 0.2 mmol CuCF₃) was added to the stirred solution of 6-benzyloxynaphthalene-2-boronic acid (0.4 mmol, 111 mg) in 1.0 mL of DMF. Solution of AgOTf (0.4 mmol, 103 mg) in DMF (1.0 mL) was added. After stirring in air for 10 min the mixture was diluted with 1M HCl, extracted with EtOAc (3×5 mL). The combined extracts were washed with brine, dried over MgSO₄ and evaporated to give the crude product of trifluoromethylation. Quantitative yield of 2-benzyloxy-6-trifluoromethylnaphthalene with respect to CuCF₃ used was observed by ¹⁹F NMR (trifluorotoluene as an internal standard). The product was purified via column chromatography (dichloromethane - petroleum ether 10:90) to give 55 mg (92%) as a colorless solid. HRMS (API): 303.0998 (calculated for C18H14OF3: 303.0997).

In a parallel run 1.0 mL of CuCF₃ solution was added to the freshly prepared stirred solution of 6-benzyloxynaphthalene-2-boronic acid (0.2 mmol, 56 mg) and AgOAc (0.2 mmol, 33 mg) in DMF (1.0 mL). After stirring in air for 10 min the mixture was quenched as described above and analysed by ¹⁹F NMR. The yield of 2-benzyloxy-6-trifluoromethylnaphthalene with respect to CuCF₃ used was 71 % (trifluorotoluene as an internal standard).

In a parallel run 1.0 mL of CuCF₃ solution was added to the freshly prepared stirred solution of 6-benzyloxynaphthalene-2-boronic acid (0.2 mmol, 56 mg) and AgOTs (0.2 mmol, 56 mg) in DMF (1.0 mL). After stirring in air for 10 min the mixture was quenched as described above and analysed by ¹⁹F NMR. Quantitative yield of 2-benzyloxy-6-trifluoromethylnaphthalene with respect to CuCF₃ used was observed (trifluorotoluene as an internal standard).

In a parallel run 1.0 mL of CuCF₃ solution was added to the freshly prepared stirred solution of 6-benzyloxynaphthalene-2-boronic acid (0.2 mmol, 56 mg) and AgNO₃ (0.2 mmol, 34 mg) in DMF (1.0 mL). After stirring in air for 10 min the mixture was quenched as described above and analysed by ¹⁹F NMR. Quantitative yield of 2-benzyloxy-6-trifluoromethylnaphthalene with respect to CuCF₃ used was observed (trifluorotoluene as an internal standard).

In a parallel run 1.0 mL of CuCF₃ solution was added to the freshly prepared stirred solution of 6-benzyloxynaphthalene-2-boronic acid (0.2 mmol, 56 mg) and AgSbF₆ (0.2 mmol, 69 mg) in DMF (1.0 mL). After stirring in air for 10 min the mixture was quenched as described above and analysed by ¹⁹F NMR. The yield of 2-benzyloxy-6-trifluoromethylnaphthalene with respect to CuCF₃ used was 92% (trifluorotoluene as an internal standard).

### Synthesis of 2-(trifluoromethyl)benzo[b]thiophene and 3-nitrotrifluoromethylbenzene:

The following compounds were prepared according to the above procedure starting from 0.3 mmol of CuCF₃: 2-(trifluoromethyl)benzo[b]thiophene (yield: 82% by ¹⁹F NMR, 72% isolated); 3-nitrotrifluoromethylbenzene (yield: 90% by ¹⁹F NMR, 66% isolated). Spectral data of these compounds were consistent with those reported in the literature [Chu, L.; Qing, F.-L. Org. Lett. 2010, 12, 5060].

### 5. Trifluoromethylation of a boronic acid without addition of neither an acid nor a borate

### Synthesis of 2-benzyloxy-6-trifluoromethylnaphthalene:

CuCl (0.5 mmol, 50 mg) and tBuOK (1.5 mmol, 170 mg) were placed into a 10-mL flask equipped with a stirring bar. The flask was sealed with a septum, flushed with nitrogen. DMF (5.0 mL) was added and the resulting colorless mixture was stirred for 20 min. A balloon filled with nitrogen was attached. Excess of CHF₃ was introduced (balloon is slightly inflated).

1.0 mL of the above solution (theoretically containing 0.1 mmol CuCF₃) was added to the stirred solution of 6-benzyloxynaphthalene-2-boronic acid (0.4 mmol, 111 mg) and AgOTf (0.4 mmol, 103 mg) in DMF (1.0 mL). After stirring in air for 10 min the mixture was diluted with 1M HCl, extracted with EtOAc (3×5 mL). The combined extracts were washed with brine, dried over MgSO₄ and evaporated to give the crude product of trifluoromethylation. Quantitative yield of 2-benzyloxy-6-trifluoromethylnaphthalene with respect to CuCF₃ used was observed by ¹⁹F NMR (trifluorotoluene as an internal standard).

In a parallel run, 1.0 mL of the above solution (theoretically containing 0.1 mmol CuCF₃) was added to the stirred solution of 6-benzyloxynaphthalene-2-boronic acid (0.4 mmol, 111 mg) and AgOTf (0.2 mmol, 51 mg) in DMF (1.0 mL). After stirring in air for 10 min the mixture was diluted with 1M HCl, extracted with EtOAc (3×5 mL). The combined extracts were washed with brine, dried over MgSO₄ and evaporated to give the crude product of trifluoromethylation. ¹⁹F NMR yield of 2-benzyloxy-6-trifluoromethylnaphthalene with respect to CuCF₃ used was 84% (trifluorotoluene as an internal standard).

### 6. Synthesis of 2-(trifluoromethyl)benzo[b]thiophene from in situ generated CHF₃ in the presence of TREAT-HF

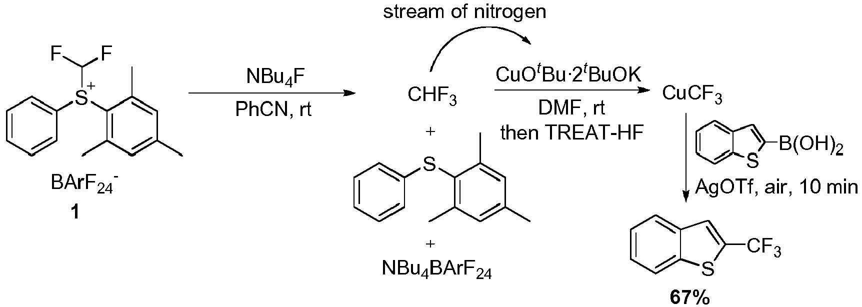

Flask 1: CuCl (0.2 mmol, 20 mg) and tBuOK (0.6 mmol, 67 mg) were put into a 10-mL Schlenck tube equipped with a stirring bar and a glass adaptor with two joints, narrow tube allowing to bubble the gas through the solution in the Schlenk tube and a stopcock in the middle. The flask was flushed with nitrogen. DMF (2.0 mL) was added via syringe. The mixture was stirred for 20 min at room temperature.

Flask 2: Tetrabutylammonium fluoride trihydrate (0.03 mmol, 10 mg) was placed into a 10-mL twin-neck flask equipped with a stirring bar and a septum. The flask was connected to the above apparatus via a second joint of the adaptor and flushed with nitrogen. PhCN (5.0 mL) was added, followed by a solution of 1˙DCM (0.03 mmol, 35 mg) in PhCN (1.0 mL).

After stirring for 5 min the stream of nitrogen was passed in the direction flask 2 to flask 1 for 7 min. Solution of TREAT-HF (0.2 mmol) in DMF (0.5 mL) was added to the flask 1. The flask 1 was opened to atmosphere, then the solution of 2-benzo[b]thienylboronic acid (0.2 mmol, 35 mg) and AgOTf (0.2 mmol, 51 mg) in DMF (1.0 mL) was added. Stream of air was passed through the resulting mixture for several seconds. After stirring for 10 min the mixture was diluted with 1M HCl, and then extracted with EtOAc (2×5 mL). The combined extracts were washed with brine, dried over MgSO₄ and evaporated to give the crude product of trifluoromethylation.

Yield of 2-(trifluoromethyl)benzo[b]thiophene by ¹⁹F NMR: 67% (trifluorotoluene as an internal standard).

### 7. Synthesis of 2-benzyloxy-6-trifluoromethylnaphthalene from in situ generated CHF₃ in the presence of B(OMe)₃

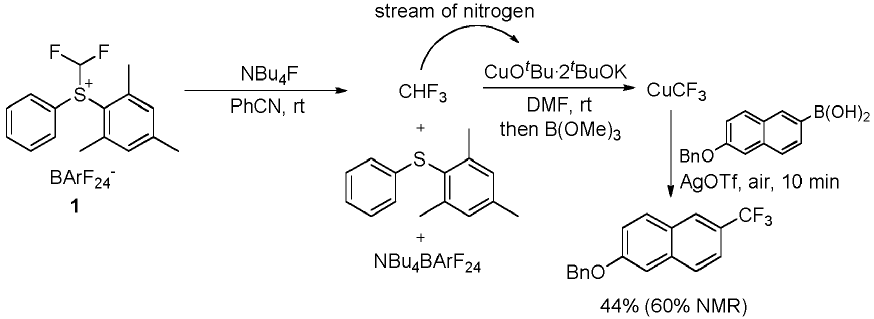

Flask 1: CuCl (0.25 mmol, 25 mg) and tBuOK (0.75 mmol, 85 mg) were put into a 10-mL Schlenck tube equipped with a stirring bar and a glass adaptor with two joints, narrow tube allowing to bubble the gas through the solution in the Schlenk tube and a stopcock in the middle. The flask was flushed with nitrogen. DMF (2.5 mL) was added via syringe. The mixture was stirred for 20 min at room temperature.

Flask 2: Compound **1**·DCM (0.05 mmol, 61 mg) was placed into a 10-mL twin-neck flask equipped with a stirring bar and a septum. The flask was connected to the above apparatus via a second joint of the adaptor. 0.3 mL of chloroform was added, then evaporated to dryness. The flask was flushed with nitrogen. PhCN (4.5 mL) was added, followed by a solution of tetrabutylammonium fluoride trihydrate (0.05 mmol, 16 mg) in PhCN (1.5 mL).

The stopcock separating the two flasks was opened. A nitrogen-filled balloon was connected to the flask 1. The stream of nitrogen was passed through the solution in the flask 2 to flask 1 for 7 min. Solution of trimethyl borate (1.0 mmol, 112 µL) in DMF (0.5 mL) was added to the flask 1. The flask 1 was opened to atmosphere, then the solution of 6-(benzyloxy)naphthaleneboronic acid (0.5 mmol, 139 mg) and AgOTf (0.5 mmol, 129 mg) in DMF (1.0 mL) was added. After stirring for 10 min the mixture was diluted with 1M HCl, and then extracted with EtOAc (2×5 mL). The combined extracts were washed with brine, dried over MgSO₄ and evaporated to give the crude product of trifluoromethylation.

Yield of 2-benzyloxy-6-trifluoromethylnaphthalene by ¹⁹F NMR: 60% (trifluorotoluene as an internal standard). Pure product was obtained after column chromatography (petroleum ether - dichloromethane 90:10). Yield: 6.7 mg (44%).

### 8. Preparation of silver-based trifluoromethylated reagents from CHF₃ and use thereof in trifluoromethylation of arylboronic acids

AgOTf (0.3 mmol, 77 mg) and tBuOK (0.6 mmol, 67 mg) were placed in a 10-mL flask equipped with a stirring bar. The flask was sealed with a septum and flushed with nitrogen. DMF (3.0 mL) was added and the resulting mixture was stirred for 10 min. Excess of CHF₃ was introduced. After stirring for 5 min B(OMe)₃ (0.6 mmol, 67 µL), then PhCF₃ (30 µL, internal standard) were added. Stirring was stopped. After the precipitate settled a sample of the solution was transferred into an NMR tube equipped with a septum under nitrogen. ¹⁹F NMR analysis indicated the presence of two species with the chemical shifts and spin-spin coupling pattern characteristic of the Ag-CF₃ fragment (integration is done using an internal standard and is based on AgOTf(100%) used; percentage is given for CF₃ groups, i.e. the quantity of the bis(CF₃)-complex is two times smaller):
- 23 ppm (pair of doublets, J(¹⁰⁹AgF) = 125 Hz) -19% (presumably AgCF₃ · DMF)
- 26 ppm (pair of doublets, J(¹⁰⁹AgF) = 100 Hz) - 127% (presumably Ag(CF₃)₂⁻)
(Overall CF₃ observed: 146%; overall trifluoromethylated Ag species observed: 83%). NMR data for trifluoromethylated silver species are consistent with those reported earlier [Eujen, R. et al. Inorg. Chem. 1997, 36, 1464].
1.0 mL of the silver-based trifluoromethylated reagent solution prepared as described above was added to a stirred mixture of 6-(benzyloxy)naphthaleneboronic acid (0.1 mmol, 28 mg), CuCl (0.05 mmol, 5 mg) and DMF (1.0 mL) in air. After stirring for 10 min the mixture was diluted with 1M HCl, and then extracted with EtOAc (2x5 mL). The combined extracts were washed with brine, dried over MgSO₄ and evaporated to give the crude product of trifluoromethylation. Yield of 2-(trifluoromethyl)benzo[b]thiophene by ¹⁹F NMR: 87% (trifluorotoluene as an internal standard).

In a parallel run, 1.0 mL of the silver-based trifluoromethylated reagent solution prepared as described earlier was added to a stirred mixture of 6-(benzyloxy)naphthaleneboronic acid (0.1 mmol, 28 mg), Cu(OTf)₂ (0.05 mmol, 18 mg) and DMF (1.0 mL) in air. After stirring for 10 min the mixture was diluted with 1M HCl, and then extracted with EtOAc (2×5 mL). The combined extracts were washed with brine, dried over MgSO₄ and evaporated to give the crude product of trifluoromethylation. Yield of 2-(trifluoromethyl)benzo[b]thiophene by ¹⁹F NMR: 83% (trifluorotoluene as an internal standard).

### 9. NMR studies of the composition of a copper-based trifluoromethylated reagent in the presence of Ag(OTf)

CuCl (0.25 mmol, 25 mg) and tBuOK (0.75 mmol, 85 mg) were placed into a 10-mL flask equipped with a stirring bar. The flask was sealed with a septum, flushed with nitrogen. DMF (2.5 mL) was added and the resulting colorless mixture was stirred for 20 min. Excess of CHF₃ was introduced. After stirring for 3 minutes trimethyl borate (1.25 mmol, 140 µL), then PhCF₃ (internal standard, 54 µL) were added.
1 mL of the above solution (theoretically containing 0.1 mmol CuCF₃) was transferred under nitrogen to another flask. Solution of AgOTf (0.1 mmol, 26 mg) in DMF (0.5 mL) was added. Dark precipitate is formed immediately. The supernatant was transferred to an NMR tube under nitrogen and analysed by ¹⁹F NMR after 2 hours. Four main species are observed (integration is done using an internal standard and is based on CuCl (100%) used; percentage is given for CF₃ groups, i.e. the quantity of bis(CF₃)-complexes is two times smaller):
- 28 ppm (broad signal) - 53% (presumably CuCF₃·DMF)
- 32 ppm (broad signal) - 4% (presumably Cu(CF₃)₂⁻)
- 23 ppm (pair of doublets, J(¹⁰⁹AgF) = 125 Hz) -19% (presumably AgCF₃·DMF)
- 26 ppm (pair of doublets, J(¹⁰⁹AgF) = 100 Hz) - 3% (presumably Ag(CF₃)₂⁻)
(Overall CF₃ observed: 79%; overall trifluoromethylated metal species observed: 76%)

In a parallel run, 1 mL of the copper-based solution as prepared in example 9 (theoretically containing 0.1 mmol CuCF₃) was transferred under nitrogen to another flask. Solution of AgOTf (0.2 mmol, 52 mg) in DMF (1.0 mL) was added. Dark precipitate is formed. The supernatant was transferred to an NMR tube under nitrogen and analysed by ¹⁹F NMR after 2 hours. The same main species as in the first case were observed:
- 28 ppm (broad signal) - 20% (presumably CuCF₃·DMF)
- 32 ppm (broad signal) - 14% (presumably Cu(CF₃)₂⁻)
- 23 ppm (pair of doublets, J(¹⁰⁹AgF) = 125 Hz) -4% (presumably AgCF₃·DMF)
- 26 ppm (pair of doublets, J(¹⁰⁹AgF) = 100 Hz) -7% (presumably Ag(CF₃)₂⁻)
(Overall CF₃ observed: 45%; overall trifluoromethylated metal species observed: 35%) NMR data for trifluoromethylated silver species are consistent with those reported earlier [Eujen, R. et al. Inorg. Chem. 1997, 36, 1464]. Assignment of copper species is based on *[*Zanardi et al. J. Am. Chem. Soc. 2011, 133 (51), 20901].
The same trifluoromethylated metal species were observed after addition of CuCl to the silver-based trifluoromethylated reagent prepared as described in entry 8.

### 10. Trifluoromethylation of anionic triol arylboronates and arylboronates

### Synthesis of 2-benzyloxy-6-trifluoromethylnaphthalene:

Lithium 2-(hydroxymethyl)-2-methylpropan-1,3-diol 6-benzyloxynaphthaleneboronate was prepared from 2-benzyloxy-6-bromonaphthalene according to the method described in [Y. Yamamoto, M. Takizawa, X.-Q. Yu, N. Miyaura; Angew. Chem. Int. Ed. 2008, 47, 928].

CuCl (0.5 mmol, 50 mg) and tBuOK (1.5 mmol, 170 mg) were placed into a 10-mL flask equipped with a stirring bar. The flask was sealed with a septum, flushed with nitrogen. DMF (5.0 mL) was added and the resulting colorless mixture was stirred for 20 min. A balloon filled with nitrogen was attached. Excess of CHF₃ was introduced (balloon is slightly inflated).

"CuCF₃" solution (0.5 mL, theoretically containing 0.05 mmol "CuCF₃") was added to a solution of lithium 2-(hydroxymethyl)-2-methylpropan-1,3-diol 6-benzyloxynaphthaleneboronate (0.10 mmol, 37 mg) in 0.5 mL DMF. After stirring in air for 10 min the mixture was diluted with 1M HCl, extracted with EtOAc (3×5 mL). The combined extracts were washed with brine, dried over MgSO₄ and evaporated to give the crude product of trifluoromethylation. Yield of 2-benzyloxy-6-trifluoromethylnaphthalene with respect to CuCF₃ used was 26% by ¹⁹F NMR (trifluorotoluene as an internal standard).

In a parallel run, "CuCF₃" solution (0.5 mL, theoretically containing 0.05 mmol "CuCF₃") was added to a freshly prepared solution of lithium 2-(hydroxymethyl)-2-methylpropan-1,3-diol 6-benzyloxynaphthaleneboronate (0.10 mmol, 37 mg) and AgOTf (0.10 mmol, 26 mg) in 0.5 mL DMF. After stirring in air for 10 min the mixture was diluted with 1M HCl, extracted with EtOAc (3×5 mL). The combined extracts were washed with brine, dried over MgSO₄ and evaporated to give the crude product of trifluoromethylation. Yield of 2-benzyloxy-6-trifluoromethylnaphthalene with respect to CuCF₃ used was 32% by ¹⁹F NMR (trifluorotoluene as an internal standard).

In a parallel run, "CuCF₃" solution (0.5 mL, theoretically containing 0.05 mmol "CuCF₃") was added to a freshly prepared solution of lithium 2-(hydroxymethyl)-2-methylpropan-1,3-diol 6-benzyloxynaphthaleneboronate (0.10 mmol, 37 mg) and B(OMe)₃ (0.20 mmol, 22 µL) in 0.5 mL DMF. After stirring in air for 10 min the mixture was diluted with 1M HCl, extracted with EtOAc (3×5 mL). The combined extracts were washed with brine, dried over MgSO₄ and evaporated to give the crude product of trifluoromethylation. Yield of 2-benzyloxy-6-trifluoromethylnaphthalene with respect to CuCF₃ used was 48% by ¹⁹F NMR (trifluorotoluene as an internal standard).

In a parallel run, "CuCF₃" solution (0.5 mL, theoretically containing 0.05 mmol "CuCF₃") was added to a freshly prepared solution of lithium 2-(hydroxymethyl)-2-methylpropan-1,3-diol 6-benzyloxynaphthaleneboronate (0.10 mmol, 37 mg), B(OMe)₃ (0.20 mmol, 22 µL) and AgOTf (0.10 mmol, 26 mg) in 0.5 mL DMF. After stirring in air for 10 min the mixture was diluted with 1M HCl, extracted with EtOAc (3×5 mL). The combined extracts were washed with brine, dried over MgSO₄ and evaporated to give the crude product of trifluoromethylation. Yield of 2-benzyloxy-6-trifluoromethylnaphthalene with respect to CuCF₃ used was 52% by ¹⁹F NMR (trifluorotoluene as an internal standard). The crude product was subjected to the column chromatography to give 6.6 mg (44%) of 2-benzyloxy-6-trifluoromethylnaphthalene.

In a parallel run, "CuCF₃" solution (0.37 mL, theoretically containing 0.037 mmol "CuCF₃") was added to a solution of pinacol 6-benzyloxynaphthaleneboronate (0.074 mmol, 27 mg) and B(OMe)₃ (0.15 mmol, 17 µL) in 0.37 mL DMF. After stirring in air for 10 min the mixture was diluted with 1M HCl, extracted with EtOAc (3×5 mL).

The combined extracts were washed with brine, dried over MgSO₄ and evaporated to give the crude product of trifluoromethylation. Yield of 2-benzyloxy-6-trifluoromethylnaphthalene with respect to CuCF₃ used was 11% by ¹⁹F NMR (trifluorotoluene as an internal standard).

In a parallel run, "CuCF₃" solution (0.37 mL, theoretically containing 0.037 mmol "CuCF₃") was added to a solution of pinacol 6-benzyloxynaphthaleneboronate (0.074 mmol, 27 mg), B(OMe)₃ (0.15 mmol, 17 µL) and AgOTf (0.074 mmol, 19 mg) in 0.37 mL DMF. After stirring in air for 10 min the mixture was diluted with 1M HCl, extracted with EtOAc (3×5 mL). The combined extracts were washed with brine, dried over MgSO₄ and evaporated to give the crude product of trifluoromethylation. Yield of 2-benzyloxy-6-trifluoromethylnaphthalene with respect to CuCF₃ used was 3% by ¹⁹F NMR (trifluorotoluene as an internal standard).

## Claims

1. A process to prepare fluoroform (CHF₃) comprising a substitution reaction between a fluoride salt and CHAF₂,
wherein A represents 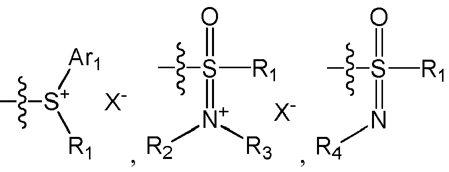 or 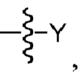 and in particular A is 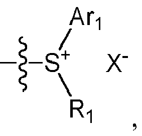 with:
■ X⁻ representing a non-nucleophile non-coordinating anion, such as BF₄⁻, PF₆⁻, SbF₆⁻, or B(Ar₂)₄⁻ with Ar₂ representing an aryl optionally substituted with one or several groups chosen from among (C₁-C₆)alkyl and (C₁-C₆)haloalkyl,
■ Y representing OSO₂R₅ or NR₆R₇R₈⁺ X⁻,
■ Ar₁ representing an aryl optionally substituted with one or several (C₁-C₆)alkyl groups,
■ R₁ representing a (C₁-C₆)alkyl group or an aryl optionally substituted with one or several (C₁-C₆)alkyl groups,
where, when R₁ represents an optionally substituted aryl group, the aryl moieties of Ar₁ and of R₁ can be bound by a single bond,
■ R₂ and R₃ representing, independently of each other, a (C₁-C₆)alkyl group,
■ R₄ representing -OS(O)₂Ar₃ with Ar₃ representing an aryl optionally substituted with one or several groups chosen from among (C₁-C₆)alkyl and (C₁-C₆)haloalkyl,
■ R₅ representing a (C₁-C₆)alkyl or (C₁-C₆)haloalkyl group, said group being optionally substituted with (C₁-C₆)alkoxy or (C₁-C₆)haloalkoxy; or an aryl optionally substituted with one or several groups chosen from among halogen, (C₁-C₆)alkyl and (C₁-C₆)haloalkyl, and
■ R₆, R₇ and R₈ representing, independently of one another, a (C₁-C₆)alkyl, aryl heteroaryl, aryl-(C₁-C₆)alkyl or (C₁-C₆)alkyl-aryl group, or
(R₆ and R₇) or (R₇ and R₈) or (R₆ and R₈) forming a heterocycle with the nitrogen atom carrying them.

2. The process according to claim 1, wherein the fluoride salt is N((C₁-C₆)alkyl)₄F or MF with M representing an alkali metal, the MF salt being preferably used in the presence of a crown ether such as 18-crown-6, and in particular the fluoride salt is NBu₄F, such as NBu₄F˙H₂O.

3. The process according to claim 1 or 2, wherein the substitution reaction is carried out in a solvent comprising a nitrile function, dichloromethane (DCM), dichloroethane (DCE), ethyl acetate (EtOAc), diethyl ether (Et₂O) or a mixture thereof; and preferably in a solvent comprising a nitrile function such as acetonitrile (ACN), benzonitrile, butyronitrile, propionitrile or a mixture thereof; and in particular in acetonitrile (ACN).

4. The process according to any one of claims 1 to 3, wherein the fluoride salt comprises fluorine-18 (¹⁸F).

5. The process according to any one of claims 1 to 4, wherein A is chosen from among: 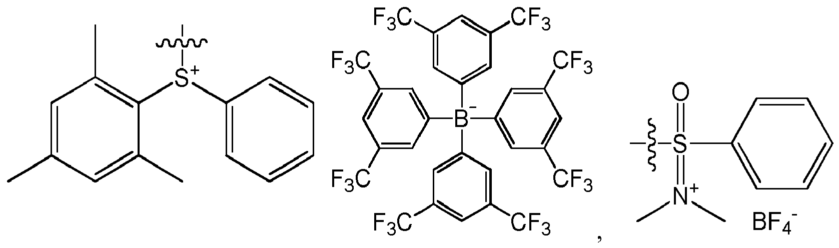 and

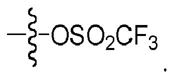

6. A process to prepare a composition comprising copper-based trifluoromethylated reagents comprising the following steps:
(i) reacting a copper (I) salt and a base to obtain a cuprating agent,
(ii) reacting the cuprating agent thus obtained with fluoroform prepared according to the process according to any one of claims 1 to 5, and
(iii) optionally adding an acid or a borate,
wherein the acid is advantageously hydrogen fluoride or a derivative or complex thereof, such as triethylamine trihydrofluoride, and the borate is B(OR₉)(OR₁₀)(OR₁₁) with R₉, R₁₀ and R₁₁, identical or different, preferably identical, representing a (C₁-C₆)alkyl group, such as a methyl.

7. The process according to claim 6, wherein the copper (I) salt is chosen from among CuCl, CuBr, CuI, CuCN, (CuOSO₂CF₃)_{˙}C₆H₆ and CuO(C₁-C₆)alkyl, and in particular is CuCl.

8. The process according to claim 6 or 7, wherein the base is an alkali metal (C₁-C₆)alkoxide, and in particular is *t*BuOK.

9. The process according to any one of claims 6 to 8, wherein the fluoroform is radio labelled with fluorine-18.

10. A composition comprising copper-based trifluoromethylated reagents radio labelled with fluorine-18 obtainable by the process according to claim 9.

11. The use of a composition comprising copper-based trifluoromethylated reagents radiolabelled with fluorine-18 according to claim 10 as a radiolabelled trifluoromethylating agent.

12. A process to prepare a composition comprising silver-based trifluoromethylated reagents comprising the following steps:
(a) reacting a silver salt and a base to obtain a silver reagent,
(b) reacting the silver reagent thus obtained with fluoroform, and
(c) optionally adding an acid or a borate,
wherein the acid is advantageously hydrogen fluoride or a derivative or complex thereof, such as triethylamine trihydrofluoride, and the borate is B(OR₉)(OR₁₀)(OR₁₁) with R₉, R₁₀ and R₁₁, identical or different, preferably identical, representing a (C₁-C₆)alkyl group, such as a methyl.

13. The process according to claim 12, wherein the silver salt is chosen from among AgOSO₂CF₃ (Ag(OTf)), AgOSO₂C₆H₄CH₃ (AgOTs), AgOSO₂CH₃, AgOAc, Ag OCOCF₃, AgCN, AgSbF₆, AgBF₄ and AgOC(CF₃)₃, and in particular is Ag(OTf).

14. The process according to claim 12 or 13, wherein the base is an alkali metal (C₁-C₆)alkoxide, and in particular is *t*BuOK.

15. The process according to any one of claims 12 to 14, wherein the fluoroform is prepared by a process according to any of claims 1 to 5.

16. A composition comprising silver-based trifluoromethylated reagents obtainable by a process according to any of claims 12 to 15.

17. The use of a mixture comprising:
- a composition comprising silver-based trifluoromethylated reagents according to claim 16, and
- a copper salt,
as a trifluoromethylating agent.

18. The use according to claim 17, wherein the copper salt is:
- a copper (I) salt such as CuCl, CuBr, CuI, Cu(CH₃CN)₄PF₆, Cu(CH₃CN)₄OTf, CuOAc, CuCN, or (CuOTf)_{2˙}C₆H₆, or
- a copper (II) salt such as Cu(OTf)₂, Cu(OAc)₂, Cu(OCOCF₃)₂ or copper (II) acetylacetonate,
and in particular, CuCl or Cu(OTf)₂.
